# EUROPÄISCHE PATENTSCHRIFT

(11) **EP 2 105 039 B1**
(45) Veröffentlichungstag und Bekanntmachung des Hinweises auf die Patenterteilung: **17.12.2014**
(21) Anmeldenummer: 08701292.8
(22) Anmeldetag: 08.01.2008
(51) Int. Cl.: H04R 1/10

(54) **Hörer mit aktiver Lärmkompensation**
Headset with active noise compensation
Écouteur à compensation active du bruit

(30) Priorität: 08.01.2007 DE 102007001980
(43) Veröffentlichungstag der Anmeldung: 30.09.2009
(73) Patentinhaber: SENNHEISER ELECTRONIC GMBH & CO. KG, 30900 Wedemark (DE)
(72) Erfinder: GRELL, Axel, 31303 Burgdorf (DE); WOLTER, Björn, 30657 Hannover (DE); SCHULZE, Elmar, 30177 Hannover (DE)
(74) Vertreter: Eisenführ Speiser
(86) Internationale Anmeldenummer: PCT/EP2008/050118
(87) Internationale Veröffentlichungsnummer: WO 2008/084035

(56) Entgegenhaltungen:
- EP-A- 1 075 164
- WO-A-94/17513
- DE-A1- 3 843 292
- US-A1- 2003 202 668

## Beschreibung

Die vorliegende Erfindung betrifft einen Hörer bzw. einen Gehörschutz.

Kopfhörer bzw. Headsets mit einer aktiven Lärmkompensation, welche beispielsweise auf einer Feedback-Regelungstechnologie beruht, sind hinlänglich bekannt.

DE 195 13 111 zeigt einen geschlossenen Kopfhörer mit einem Mikrofon und einem Wiedergabewandler sowie einer Kompensationsschaltung zur Schallkompensation. In dem Kopfhörer ist ferner ein Ausgleichselement vorgesehen, welches dazu dient, die Schalleintrittsöffnung des Mikrofons zu überdecken, um die Wirkung von Reflexionen am Ohr zu verringern. Das Ausgleichselement weist eine Platte mit auf den Wiedergabewandler gerichteten Vorsprüngen auf. Das Mikrofon wird von einer Hülse mit einer umlaufenden Wulst aufgenommen.

DE 693 15 792 T2 zeigt einen Fernsprechhandapparat mit einer Geräuschunterdrückungsfunktion. Der Handapparat weist an einem Hörerende einen elektroakustischen Wandler, ein Mikrofon zur Aufzeichnung von Störschall, eine aktive Lärmkompensationseinheit und ein Gehäuse auf. Das Mikrofon zur Aufzeichnung von Störschall ist durch ein Mikrofongehäuse und eine Hörmuschel umgeben.

DE 38 43 292 A1 zeigt eine Hörvorrichtung für Kopfhörer. Die Hörvorrichtung weist einen elektroakustischen Wandler und eine Resonanzwand auf.

Weitere Beispiele von Kopfhörern bzw. Headsets mit aktiver Lärmkompensation sind aus den Dokumenten EP 1 075 164 A, US 2003/202 668 A1 und WO 94/17 513 A bekannt.

Da die menschliche Ohrmuschel bei jedem Menschen individuell ausgestaltet ist, sind die Reflexionen am Ohr bei jedem Menschen anders. Durch das Ausgleichselement kann somit eine definierte Schallreflexion für das Mikrofon vorgesehen werden.

Bei derartigen Kopfhörern mit aktiver Lärmkompensation bzw. Störschallkompensation kann die Regelschleife für die aktive Lärmkompensation unter bestimmten Bedingungen jedoch instabil werden und sich aufschwingen. Dies kann insbesondere dann auftreten, wenn das Ohr oder Teile des Ohrs ein sehr kleines Volumen bilden, wobei sich sowohl das Mikrofon als auch der Wiedergabewandler in diesem kleinen Volumen befinden.

Es ist somit eine Aufgabe der vorliegenden Erfindung, einen Kopfhörer bzw. ein Headset oder einen Gehörschutz vorzusehen, welcher Instabilitäten bei der aktiven Lärmkompensation zumindest verringert.

Diese Aufgabe wird durch einen Kopfhörer gemäß Anspruch 1 bzw. durch ein Headset gemäß Anspruch 2 gelöst.

Somit wird ein Hörer mit einem elektroakustischen Wiedergabewandler, einem Mikrofon zur Aufzeichnung von Störschall und mit einer aktiven Lärmkompensationseinheit zum Kompensieren von Lärm basierend auf dem durch das Mikrofon aufgezeichneten Störschall vorgesehen. Der Hörer weist ein Gehäuse zur Aufnahme des Mikrofons und des elektroakustischen Wiedergabewandlers auf. Das Gehäuse weist einen ersten Gehäuseabschnitt auf, welcher zumindest das Mikrofon derart umschließt, dass ein definiertes akustisches Volumen erhalten wird.

Die Erfindung beruht auf dem Gedanken, ein akustisches Volumen in einem Gehäuse eines Hörers, insbesondere eines Kopfhörers vorzusehen, welches durch den Benutzer bzw. durch die Körpergeometrie des Benutzers nicht verändert werden kann. Zusammen mit dem Regelungsmikrofon, dem Schallwiedergabewandler und der Elektronik für die aktive Lärmkompensation kann somit ein stabiles Regelungssystem gebildet werden. Das definierte akustische Volumen wird durch mindestens ein Loch an das Ohrvolumen angebunden. Ferner wird eine Stoffgaze auf den das akustische Volumen abgrenzenden Abschnitt aufgebracht.

Somit kann ein definiertes akustisches Volumen um das Regelungsmikrofon herum ausgebildet werden, um ggf. auftretende Instabilitäten sowie ein Rückkopplungspfeifen zu vermeiden.

Die Vorteile und Ausführungsbeispiele der Erfindung werden nachstehend unter Bezugnahme auf die Zeichnung näher erläutert.
- Fig. 1: zeigt eine isometrische Ansicht eines Gehäuses für einen Wiedergabewandler eines Hörers,
- Fig. 2: zeigt eine Draufsicht auf ein Gehäuse eines Wiedergabewandlers,
- Fig. 3: zeigt eine isometrische Ansicht des Gehäuses von Fig. 2, und
- Fig. 4: zeigt eine Schnittansicht Schnitt A-A des Gehäuses von Fig. 2.

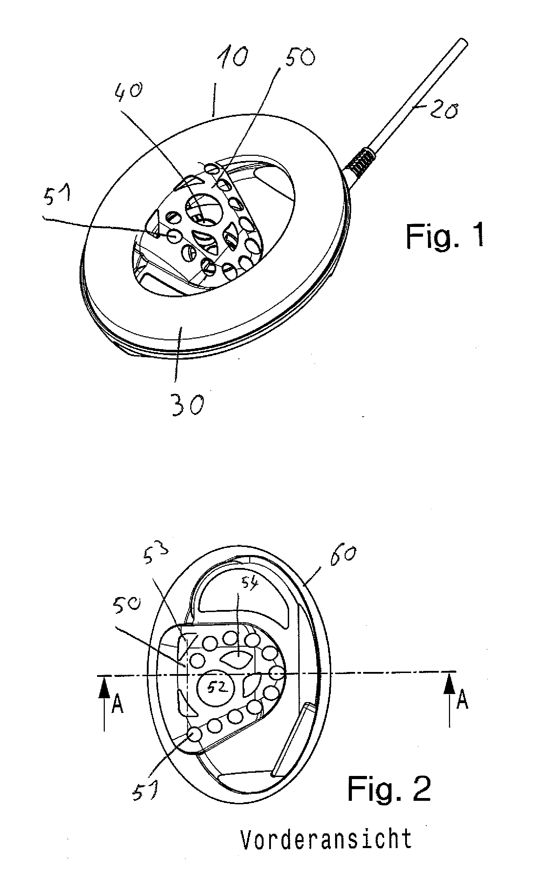

Fig. 1 zeigt eine isometrische Ansicht eines Gehäuses eines Wiedergabewandlers eines Hörers, insbesondere eines Kopfhörers. In Fig. 1 ist somit ein Gehäuse 10 eines Wiedergabewandlers eines Kopfhörers mit einem Kabel 20 gezeigt. An dem Gehäuse 10 ist ein Ohrpolster 30 angebracht. Das Gehäuse 10 weist ferner einen Wiedergabewandler und ein Mikrofon 40 auf. Der durch das Mikrofon 40 aufgezeichnete Schall wird einer aktiven Lärmkompensationseinheit zugeführt, um den aufgezeichneten Störschall von dem wiederzugebenden Audiosignal zu subtrahieren, so dass eine aktive Lärmkompensation erreicht werden kann. In dem Gehäuse 10 ist ferner ein erster Gehäuseabschnitt 50 vorgesehen, welcher dazu dient, ein definiertes akustisches Volumen um das Mikrofon 40 herum auszubilden. Der erste Gehäuseabschnitt 50 kann eine Vielzahl von Löchern 51 aufweisen. Der erste Gehäuseabschnitt 50 kann einstückig mit dem Gehäuse 10 verbunden sein.

Fig. 2 zeigt eine Draufsicht auf ein Gehäuseteil 60 des Gehäuses von Fig. 1. Hierbei ist der erste Gehäuseabschnitt 50 an bzw. mit dem Gehäuseteil 60 befestigt. Der erste Gehäuseabschnitt 50 kann dabei einstückig mit dem Gehäuseteil 60 ausgestaltet bzw. geformt sein. Alternativ dazu kann der erste Gehäuseabschnitt 50 an dem Gehäuseteil 60 befestigt oder eingeklebt werden.

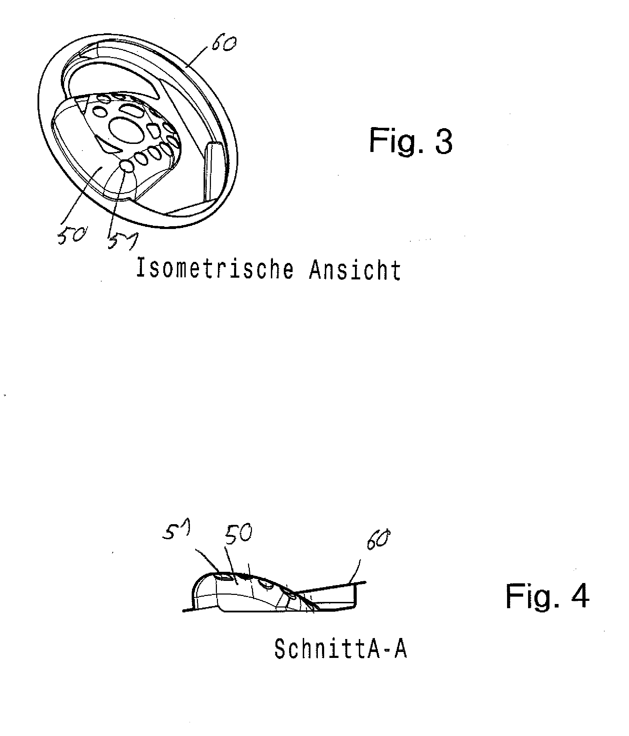

Fig. 3 zeigt eine isometrische Ansicht des Gehäuseteils 60 von Fig. 2.

Fig. 4 zeigt eine Schnittansicht entlang des Schnitts A-A von Fig. 2. Hier wird sowohl das Gehäuseteil 60 als auch der erste Gehäuseabschnitt 50 gezeigt. Der erste Gehäuseabschnitt 50 weist dabei zumindest teilweise eine vorgegebene Krümmung auf.

Durch das Vorsehen des ersten Gehäuseabschnitts 50, welches bezüglich des Regelungsmikrofons 40 ein definiertes akustisches Volumen ausbildet, kann eine Instabilität in der Regelungsschleife aufgrund einer Veränderung des akustischen Volumens im Bereich des Regelungsmikrofons verhindert werden. Damit kann ebenfalls das unerwünschte Rückkopplungspfeifen verringert werden. Das durch den ersten Gehäuseabschnitt 50 definierte akustische Volumen mit dem sich darin befindlichen Regelungsmikrofon und dem Schallwiedergabewandler und der Elektronik der aktiven Lärmkompensation stellt somit ein stabiles Regelungssystem dar.

Der erste Gehäuseabschnitt 50 weist vorzugsweise mindestens ein Loch 51 auf, welches dazu dient, das durch den ersten Gehäuseabschnitt 50 definierte akustische Volumen an das Ohrvolumen anzubinden. Zusätzlich dazu kann eine Stoffgaze im Bereich des ersten Gehäuseabschnitts 50 vorgesehen werden.

In dem ersten Gehäuseabschnitt 50 sind eine Anzahl von kreisrunden Löchern 51 sowie ein größeres kreisrundes Loch 52 im mittleren Bereich des ersten Gehäuseabschnitts 50 angeordnet. Im Verbindungsbereich zwischen dem Gehäuseteil 60 und dem ersten Gehäuseabschnitt 50 sind zwei im Wesentlichen dreieckförmige Löcher 53 ausgebildet, wobei eine Ecke des Loches abgerundet ausgestaltet ist. Im mittleren Bereich des ersten Gehäuseabschnitts sind ferner zwei Löcher 54 ausgebildet.

Der erste Gehäuseabschnitt 50 kann sowohl zur Aufnahme des Mikrofons als auch zur zumindest teilweisen Aufnahme des elektroakustischen Wiedergabewandlers dienen.

Gemäß einem weiteren Ausführungsbeispiel wird ein Headset basierend auf dem oben beschriebenen Hörer vorgesehen. Dazu muss lediglich ein weiteres Mikrofon zur Aufzeichnung von Sprachsignalen vorgesehen werden.

Der oben beschriebene Hörer kann z.B. als ein Kopfhörer oder ein Ohrhörer ausgestaltet sein.

## Patentansprüche

1. Kopfhörer, mit
einem elektroakustischen Wiedergabewandler,
einem Mikrofon (40) zur Aufzeichnung von Störschall,
einer aktiven Lärmkompensationseinheit zur Kompensierung von Lärm basierend auf dem durch das Mikrofon (40) aufgezeichneten Störschall, und
einem Gehäuse (10) zur Aufnahme des Mikrofons (40) und des elektroakustischen Wiedergabewandlers,
wobei das Gehäuse (10) einen ersten Gehäuseabschnitt (50) aufweist, welches zumindest das Mikrofon (40) derart umschließt, dass ein definiertes akustisches Volumen erhalten wird, welches durch den Benutzer nicht veränderbar ist, wobei eine Stoffgaze über dem ersten Gehäuseabschnitt (50) angebracht wird,
wobei der Wiedergabewandler zumindest teilweise von dem ersten Gehäuseabschnitt (50) umgeben ist,
wobei der erste Gehäuseabschnitt (50) mindestens ein Loch (51, 52, 53, 54) aufweist, um das durch den ersten Gehäuseabschnitt (50) definierte akustische Volumen an ein Ohrvolumen anzubinden,
wobei das Gehäuse (10) ein Gehäuseteil (60) aufweist, das einstückig mit dem ersten Gehäuseabschnitt (50) ausgestaltet ist.

2. Headset, mit
einem ersten Mikrofon zur Aufzeichnung von Sprachsignalen,
einem elektroakustischen Wiedergabewandler,
einem zweiten Mikrofon (40) zur Aufzeichnung von Störschall,
einer aktiven Lärmkompensationseinheit zur Kompensierung von Lärm basierend auf dem durch das zweite Mikrofon (40) aufgezeichneten Störschall, und
einem Gehäuse (10) zur Aufnahme des zweiten Mikrofons (40) und des elektroakustischen Wiedergabewandlers,
wobei das Gehäuse (10) einen ersten Gehäuseabschnitt (50) aufweist, welches zumindest das zweite Mikrofon (40) derart umschließt, dass ein definiertes akustisches Volumen erhalten wird, welches durch den Benutzer nicht veränderbar ist, wobei eine Stoffgaze über dem ersten Gehäuseabschnitt (50) angebracht wird,
wobei der Wiedergabewandler zumindest teilweise von dem ersten Gehäuseabschnitt (50) umgeben ist,
wobei der erste Gehäuseabschnitt (50) mindestens ein Loch (51, 52, 53, 54) aufweist, um das durch den ersten Gehäuseabschnitt (50) definierte akustische Volumen an ein Ohrvolumen anzubinden,
wobei das Gehäuse (10) ein Gehäuseteil (60) aufweist, das einstückig mit dem ersten Gehäuseabschnitt (50) ausgestaltet ist.

## Claims

1. Headset, having
an electroacoustic reproduction converter,
a microphone (40) for recording ambient noise,
an active noise compensation unit for compensating for noise based on the ambient noise recorded by the microphone (40), and
a housing (10) for receiving the microphone (40) and the electroacoustic reproduction converter,
the housing (10) having a first housing portion (50) which at least surrounds the microphone (40) in such a manner that a defined acoustic volume which cannot be changed by the user is achieved, a material gauze being fitted over the first housing portion (50),
the reproduction converter being at least partially surrounded by the first housing portion (50),
the first housing portion (50) having at least one hole (51, 52, 53, 54) in order to link the acoustic volume defined by the first housing portion (50) to an ear volume,
the housing (10) having a housing portion (60) which is constructed integrally with the first housing portion (50).

2. Headset, having
a first microphone for recording speech signals,
an electroacoustic reproduction converter,
a second microphone (40) for recording ambient noise,
an active noise compensation unit for compensating for noise based on the ambient noise recorded by the second microphone (40), and
a housing (10) for receiving the second microphone (40) and the electroacoustic reproduction converter,
the housing (10) having a first housing portion (50) which at least surrounds the second microphone (40) in such a manner that a defined acoustic volume which cannot be changed by the user is achieved, a material gauze being fitted over the first housing portion (50),
the reproduction converter being at least partially surrounded by the first housing portion (50),
the first housing portion (50) having at least one hole (51, 52, 53, 54) in order to link the acoustic volume defined by the first housing portion (50) to an ear volume,
the housing (10) having a housing portion (60) which is constructed integrally with the first housing portion (50).

## Revendications

1. Ecouteur, comprenant
un émetteur électroacoustique,
un microphone (40) pour l'enregistrement de bruit parasite,
une unité active de compensation du bruit pour compenser le bruit en se basant sur le bruit parasite enregistré par le microphone (40), et
un boîtier (10) pour recevoir le microphone (40) et l'émetteur électroacoustique,
dans lequel le boîtier (10) présente une première section de boîtier (50), qui renferme au moins le microphone (40) de telle manière qu'un volume acoustique défini est obtenu, qui ne peut être modifié par l'utilisateur, une gaze de tissu étant appliquée sur la première section de boîtier (50) ,
dans lequel l'émetteur est entouré au moins en partie par la première section de boîtier (50),
dans lequel la première section de boîtier (50) présente au moins un trou (51, 52, 53, 54) afin de rattacher le volume acoustique défini par la première section de boîtier (50) à un volume de l'oreille,
dans lequel le boîtier (10) présente une partie de boîtier (60), qui est configurée d'une seule pièce avec la première section de boîtier (50).

2. Casque d'écoute, comprenant
un premier microphone pour enregistrer des signaux vocaux,
un émetteur électroacoustique,
un deuxième microphone (40) pour l'enregistrement de bruit parasite,
une unité active de compensation du bruit pour compenser le bruit sur la base du bruit parasite enregistré par le deuxième microphone (40), et
un boîtier (10) pour recevoir le deuxième microphone (40) et l'émetteur électroacoustique,
dans lequel le boîtier (10) présente une première section de boîtier (50), qui renferme au moins le deuxième microphone (40) de telle manière qu'un volume acoustique défini est obtenu, qui ne peut être modifié par l'utilisateur, une gaze de tissu étant appliquée sur la première section de boîtier (50),
dans lequel l'émetteur est entouré au moins en partie par la première section de boîtier (50),
dans lequel la première section de boîtier (50) présente au moins un trou (51, 52, 53, 54) afin de rattacher le volume acoustique défini par la première section de boîtier (50) à un volume de l'oreille,
dans lequel le boîtier (10) présente une partie de boîtier (60), qui est configurée d'une seule pièce avec la première section de boîtier (50).
